# EUROPEAN PATENT APPLICATION

(11) **EP 4 368 218 A1**
(43) Date of publication of application: **15.05.2024**
(21) Application number: 22206048.5
(22) Date of filing: 08.11.2022
(51) Int. Cl.: A61M 1/00, A61M 3/02

(54) **SYSTEM FOR FLUSHING A WOUND AND/OR A BODY CAVITY, IN PARTICULAR THE PERICARDIAL CAVITY**

(71) Applicant: Haermonics BV, 5616 LZ Eindhoven (NL)
(72) Inventor: Koch, Leonardus Jozef Lucas Maria, 5616 LZ Eindhoven (NL); Koolbergen, David Robert, 5616 LZ Eindhoven (NL); Markus, Willem Paul, 5616 LZ Eindhoven (NL)
(74) Representative: DTS Patent- und Rechtsanwälte Schnekenbühl und Partner mbB

(57) **Abstract**

The invention provides a flushing system (1, 200, 300, 400, 500) configured to flush a wound and/or a body cavity, in particular the pericardial cavity (PC), of a patient, wherein the system (1, 200, 300, 400, 500) comprises: an infusion liquid outlet (2) to connect a first tube (4) having an infusion liquid lumen to guide a flow of infusion liquid from the system (1, 200, 300, 400, 500) to the wound and/or the body cavity, in particular the pericardial cavity (PC), an effusion liquid inlet (6) to connect to a second tube (8) having an effusion liquid lumen to guide the effusion liquid flow from the wound and/or the body cavity, in particular the pericardial cavity (PC), to the system (1, 200, 300, 400, 500), a buffer container (10) having a buffer volume and comprising a buffer inlet (12) to receive effusion liquid from the wound and/or the body cavity, in particular the pericardial cavity (PC), and a first buffer outlet (14) to output the received effusion liquid, a flow rate control system to control the flow rate of the effusion liquid output from the buffer container (10), wherein the flow rate control system comprises: a control unit (16) to provide one or more control signals, a first pump device (18) for pumping effusion liquid from the buffer container (10) to one or more effusion liquid container (20) at an effusion liquid flow rate, wherein the effusion liquid flow rate is adjustable by the control signals of the control unit (16), wherein the buffer container (10) comprises a clot trap (22) configured for preventing clots included in the effusion liquid being received from the wound and/or the body cavity, in particular the pericardial cavity (PC), from exiting the buffer container (10) via the first buffer outlet (14), and/or wherein the buffer container (10) is configured for air-liquid-separation, and/or configured with a safety bypass, and/or configured for receiving liquid gushes.

## Description

The present invention relates to a flushing system configured for postoperative flushing of a wound and/or a body cavity, in particular the pericardial cavity of a patient. In particular, the present invention relates to a flushing system for flushing a/the wound and/or a/the body cavity, in particular the pericardial cavity of a patient as a postoperative treatment in order to reduce postoperative blood loss, reduce the accumulation of blood and clots in the wound and/or the body cavity, in particular the pericardial cavity, reduce the risk of cardiac tamponade, and reduce the reduction of blood loss and clotting related complications.

Excessive postoperative blood loss which might amount to more than 2 I per 24 hours or more than 200 ml per hour is a known cause of complications at cardiac surgery. Reoperation/re-exploration for bleeding is a strong independent risk factor for adverse outcome following cardiac surgery with higher mortality and morbidity rates. In addition, postoperative bleeding requiring multiple transfusions and surgical re-exploration is associated with higher costs, increased sternal wound infection, and transfusion-related infection.

To evacuate blood from the pericardial cavity - and if necessary pleural cavities - chest tubes are left postoperative. However, when blood loss is excessive or when clots start to develop more rapidly, the drains often fail in their function to evacuate all accumulated blood. Stasis of clots and blood in the pericardial (and/or pleural) cavity may lead to high fibrinolytic activity, maintenance of blood loss and in some cases to cardiac tamponade and, as a possible result, to excessive filling pressure of the heart.

A pericardial flushing system may be specifically designed to clean the pericardial space after cardiac surgery. The system works by flushing the pericardial cavity with a saline (or other) solution, thereby lowering the viscosity and the hematocrit value of the blood present in the cavity and at the same time preventing the formation of larger clots. Cleaning the pericardial space by flushing will prevent clogging of the chest drains and the consequent accumulation of blood and clots in the pericardial space. This will lead to less postoperative bleeding and diminish the risk for provoking an acute cardiac tamponade. In this respect, in order to obtain the amount of blood loss while flushing, the system comprises sensor means, in particular a hematocrit sensor, for providing the basis for obtaining the amount of blood loss. However, the accuracy and/or precision of measurements conducted by such sensor means may also depend on further elements, such as clots and/or air, being present in the diluted pericardial blood effusion liquid and being able to affect said measurements. Furthermore, the flow of the liquid to be measured may also affect the accuracy of the results of said measurements, wherein a steady flow is usually preferred. Hence, there is a need for providing effusion liquid received from the pericardial cavity in a more suitable / optimal condition for further processing, e.g., for analysis.

An example of prior art with respect to a pericardial flushing system can be found, e.g., in WO 2015/086857 A1.

Accordingly, it is an object of the invention to provide a flushing system configured to flush a wound and/or a body cavity, in particular the pericardial cavity, of a patient, which can be operated in a more accurate manner.

This is achieved by a flushing system configured to flush a wound and/or a body cavity, in particular the pericardial cavity, of a patient as defined in claim 1.

In this respect, a flushing system configured to flush a wound and/or a body cavity, in particular the pericardial cavity, of a patient is provided, wherein the system comprises:
- an infusion liquid outlet to connect a first tube having an infusion liquid lumen to guide a flow of infusion liquid from the system to the wound and/or the body cavity, in particular the pericardial cavity,
- an effusion liquid inlet to connect to a second tube having an effusion liquid lumen to guide the effusion liquid flow from the wound and/or the body cavity, in particular the pericardial cavity, to the system,
- a buffer container having a buffer volume and comprising a buffer inlet to receive effusion liquid from the wound and/or the body cavity, in particular the pericardial cavity, and a first buffer outlet to output the received effusion liquid,
- a flow rate control system to control the flow rate (e.g., the volume) of the effusion liquid output from the buffer container, wherein the flow rate control system comprises:
   - a control unit to provide one or more control signals,
   - a first pump device for pumping effusion liquid from the buffer container to (e.g., towards) one or more effusion liquid container at an effusion liquid flow rate (e.g., with a certain volume), wherein the effusion liquid flow rate (e.g., the volume) is adjustable by the control signals of the control unit,
- wherein the buffer container comprises an (optional) clot trap configured for preventing clots included in the effusion liquid being received from the wound and/or the body cavity, in particular the pericardial cavity, from exiting the buffer container via the first buffer outlet, and/or wherein the buffer container is configured for air-liquid-separation, and/or configured with a safety bypass, and/or configured for receiving liquid gushes.

The invention is particularly based on the idea that the buffer container may provide different functions, which may dependently or independently be fulfilled and/or occur, wherein, firstly, clots may be trapped by said clot trap, secondly, air and liquid may be separated from each other, thirdly, sudden gushes of effusion liquid may be collected, and, fourthly, effusion liquid may be provided for discreet measurements. Further, due to air-liquid-separation and/or the safety bypass, a constant vacuum may be applicable to the wound and/or the body cavity, in particular the pericardial cavity, for draining. In this respect, on the one hand, the effusion liquid is buffered before being guided further for subsequent proceedings (e.g., analysis) and, on the other hand, the effusion liquid is freed from clots. This is achieved by providing a/the buffer container comprising a/the clot trap. The clot trap prevents clots included in the effusion liquid from being guided further. The clot trap may be a mechanical means. Due to buffering of the effusion liquid, a more steady flow with a certain (e.g., known) volume leaving the buffer container for further proceedings may be achieved, to thereby allow subsequent sensor means to conduct more precise measurements. Additionally, due to the removal of the clots, subsequent sensor means may also be allowed to conduct more precise measurements. Furthermore, the buffer container may serve as a safety means with respect to pressure. In this respect, the buffer volume of the buffer container may store liquid which might (otherwise) have caused back pressure / afflux in the system (e.g., towards the effusion liquid inlet). Further, the buffer container may vent the effusion liquid such that air may be discharged therefrom in order not to influence a subsequent analysis / measurements. In other words, the buffer container may be configured to separate air from liquid, in particular the effusion liquid.

The buffer container may substantially have a cylindrical shape extending along a central axis. However, it should be understood that any other shape may be possible in this regard. The buffer container may comprise a top end and a bottom end. The buffer container may comprise a top wall, a side wall and a bottom wall. The buffer inlet may be disposed closer to the top wall than to the bottom wall. The first buffer outlet may be disposed closer to the bottom wall than to the top wall. The buffer inlet may be arranged in the top wall or the side wall. The first buffer outlet may be arranged in the bottom wall or the side wall. Hence, the buffer inlet may be arranged at a higher level than the first buffer outlet with respect to the gravitational direction. When the buffer container is incorporated, e.g., mounted, in the system, the central axis substantially extends along the gravitational direction.

The flushing system may be configured to provide approximately 500 ml of infusion liquid per hour, i.e., the infusion inflow may be approximately 500 ml per hour. Hence, the buffer volume may be sized accordingly. However, the system and, accordingly, the buffer volume may be configured to handle sudden gushes of 400 ml and/or 500 ml of effusion liquid per 5 min.

The clot trap may comprise a sieve structure being disposed between the buffer inlet and the first buffer outlet of the buffer container, to thereby prevent clots from passing through the first buffer outlet. The clot trap may be integrally formed with the buffer container (e.g., on the side wall or the bottom wall of the buffer container). Alternatively, the clot trap may be mounted to the buffer container via fixing means. The sieve structure may comprise a plurality of openings formed therein. The openings may be configured to stop clots from passing through the sieve structure.

The clot trap may have a conical shape having a base facing towards the first buffer outlet and an apex facing the buffer inlet. It is also possible, that the clot trap may have other shapes, e.g., a truncated cone shape, a dome shape, a flat shape etc. In particular, due to said shape of the clot trap, certain amounts of clots may be trapped in the clot trap without blocking the clot trap.

The buffer container may further comprise at least one level sensor for obtaining at least one filling level of the buffer volume.

The buffer container may further comprise a first level sensor and a second level sensor for obtaining a first filling level and a second filling level of the buffer volume, respectively, wherein the second level sensor may be arranged at a higher level than the first level sensor with respect to the gravitational direction.

A level sensor (e.g., the above level sensors) may be configured to obtain a (the) filling level, for example, mechanically or optically. However, any other suitable sensor may also be applicable (such as capacitive sensors, resistive sensors, ultrasonic sensors etc.).

The control unit may provide at least one of the control signals on the basis of the obtained filling level of the buffer volume and/or a buffer container level, to thereby control the first pump device and adjust the effusion liquid rate (e.g., pumped volume) from the buffer container. Hence, the buffer container may collect effusion liquid until the first pump device is allowed to pump. Accordingly, a steady flow of effusion liquid may be generated in a controlled manner. As a consequence, subsequent analysis means may perform precise measurements in/at the effusion liquid. The control unit may be configured to control the first pump device in order to pump a predetermined, i.e., a known, volume on the basis of said control signals. For example, the control unit may be configured to control the first pump device in order to pump a volume of, e.g., 30 ml, which may be referred to as a batch. However, any other volume may be pumped on the basis of the control signals of the control unit. Accordingly, the first pump device may be operated in a discontinuous manner by the control unit. Additionally or alternatively, the first pump device may be controlled by a control signal being based on an amount of outflow liquid.

The first pump device may comprise a peristaltic pump. It may additionally or alternatively be conceivable, that the first pump device may comprise a volumetric pump, a membrane pump, an impeller pump and/or a syringe pump device including a syringe pump and, optionally, at least two valves. In particular, the first pump device may be any suitable pump device for effusion liquid (with or without valves, e.g., separate valves), and is preferably of a type that allows pumping at a relative accurate flow rate (e.g., that allows accurately pumping of (certain) volumes). The peristaltic pump or the syringe pump, for example, may be configured for gently conveying of sensitive material such as blood cells, which could be destroyed by rapidly moving or rotating elements, which might be part of other kinds of pumps. The peristaltic pump may perform a continuous and/or a discontinuous pumping action. The syringe pump may perform a discontinuous pumping action, wherein the pumping action of the syringe pump has a suction step and a discharging step. In the suction step, a first valve disposed upstream from the syringe pump is opened and a second valve disposed downstream from the syringe pump is closed, and, in the discharging step, the first valve is closed and the second valve is opened.

The system may further comprise the (above-mentioned) one or more effusion liquid containers to receive effusion liquid out of the buffer container. The one or more effusion liquid containers may be connectable or connected to the buffer container (e.g., via the first buffer outlet).

The flushing system may further comprise one or more suction devices to draw effusion liquid out of the wound and/or the body cavity, in particular the pericardial cavity. The one or more suction devices may be arranged to create a relative low pressure in the one or more effusion liquid containers to receive effusion liquid so that effusion liquid is drawn towards the one or more effusion liquid containers. For example, the one or more effusion liquid containers may be connectable or connected to an intensive care unit (ICU) vacuum wall connector or the like (e.g., any other suitable vacuum providing unit/system and/or any other suitable custom made vacuum unit), wherein the ICU vacuum wall connector may be configured to create said (relative) low pressure and may thereby act as said one or more suction devices. Additionally or alternatively, a system's own vacuum unit may also be provided, in particular for the above-described purpose.

The buffer container may further comprise a second buffer outlet being arranged at a higher level than the first buffer outlet with respect to the gravitational direction and being configured to output effusion liquid and/or air, to thereby allow an air-liquid-separation and/or allow to function as the safety bypass. Further, the second buffer outlet may be arranged at a lower level than the buffer inlet with respect to the gravitational direction. The second buffer outlet may be connectable or connected to the one or more effusion liquid containers. For example, the second buffer outlet may be connectable or connected to the one or more effusion liquid containers in a direct and/or an always open manner.

The second buffer outlet may be disposed on an opposite side of the clot trap with respect to the first buffer outlet. Hence, the second buffer outlet may be disposed on the same side as the buffer inlet.

The second buffer outlet may act as a safety overflow and/or a safety bypass from the buffer container, to thereby provide an open connection to the one or more effusion liquid containers for safety reasons (e.g., for preventing back pressure /afflux). Further, the second buffer outlet may also provide an exit path for air being separated from the effusion liquid in the buffer container. The separated air may be sucked into the one or more suction devices, e.g., the ICU vacuum wall connector and/or a system's own vacuum unit. In other words, the separated air may be sucked via the one or more effusion liquid containers into the ICU vacuum wall connector and/or into the system's own vacuum unit. Hence, due to the lack of air in the effusion liquid, the effusion liquid may subsequently be analyzable or analyzed in a more accurate and reliable manner.

The system may further comprise a hematocrit sensor configured to measure the hematocrit value of the effusion liquid for obtaining a blood loss volume and/or a blood loss flow rate from the wound and/or the body cavity, in particular the pericardial cavity. The hematocrit sensor may be configured to optically measure said hematocrit value based on scattering and absorption of light emitted into the effusion liquid.

By a hematocrit analysis and/or measurement of the outflow fluids, i.e., effusion liquid, the exact amount of blood loss may be calculated more accurately. This may improve clinical decision making with regard to optimal timing of re-exploration and blood transfusion policy.

The hematocrit sensor may be disposed for conducting measurements in the effusion liquid pumped from the buffer container to the one or more effusion liquid containers. Hence, the hematocrit sensor may be arranged downstream from the buffer container and upstream from the one or more effusion containers. Due to said arrangement of the hematocrit sensor, precise hematocrit measurements may be conducted without negative effects of clots and/or air in the effusion liquid. Further, since the first pump device may be controlled with respect to the volume of liquid to be pumped by the control unit, the measured hematocrit value may be set in correlation with the pumped volume, in which the hematocrit value is measured by said sensor. Hence, the blood loss volume and/or the blood loss flow rate from the wound and/or the body cavity, in particular the pericardial cavity, may be measured in an easy, reliable and/or accurate manner.

It may also be conceivable, that the system may further comprises a unit for blood loss measurements and/or patient systematic measurements, which is configured for obtaining the blood loss volume and/or the blood loss flow rate from the wound and/or the body cavity, in particular the pericardial cavity (PC), and/or may be connectible to such a unit.

The system may comprise a second pump device for pumping infusion liquid to the infusion liquid outlet at an infusion liquid flow rate, wherein the infusion liquid flow rate may be adjustable by the control signals of the control unit. The second pump device may be any suitable pump device for infusion liquid, and is preferably of a type that allows pumping at a relative accurate flow rate (e.g., at 2% accuracy or better at a nominal flow rate of, e.g., 500 ml/hour). In this respect, the second pump device may be a peristaltic pump.

It may additionally or alternatively be possible, that the second pump device may comprise a volumetric pump, a membrane pump, an impeller pump and/or a syringe pump device including a syringe pump and, optionally, at least two valves. The second pump device may be connectable or connected to one or more infusion liquid containers.

Hence, optionally, the system may comprise above-mentioned one or more infusion liquid containers containing infusion liquid, for example a number of bags containing saline fluid, such as NaCl 0,9%, as known in the art. The one or more containers may be connectable or connected to the second pump device being configured to pump the infusion liquid with a desired flow rate towards the infusion liquid outlet.

The infusion liquid flow rate of the second pump device may be adjustable by the control unit so that the control unit may control the flow rate of infusion liquid that is pumped to the wound and/or the body cavity, in particular the pericardial cavity. For example, the infusion liquid flow rate of the second pump device may be adjustable by the control unit in an open loop and/or closed loop manner, in particular, with respect to an operator/user and/or the system itself.

The system may further comprise a pressure sensor being positioned inside or in connection with the first tube, the second tube, the buffer container or the wound and/or the body cavity, in particular the pericardial cavity, and being configured for obtaining the pressure in the wound and/or the body cavity, in particular the pericardial cavity. In other words, the pressure sensor may be configured to provide a pressure signal representative for the pressure in the wound and/or the body cavity, in particular the pericardial cavity. The obtained pressure may be provided to the control unit and/or a pressure control unit, which may be part of the control unit or a separate unit of the system and which may be configured to adjust (e.g., even stop) the infusion liquid flow rate to keep the pressure (i.e., for example, the pressure signal) within desired pressure limits so as to avoid acute cardiac tamponade. Further, the pressure measurement may allow detecting a/the chance for an upcoming tamponade, and warning the clinical staff about this. Optionally, the inflow fluid may act as conductor, e.g., conductor means, of or for the measurement of the pressure in the body cavity, in particular the pericardial cavity.

The system may further comprise a heater device configured to heat the infusion liquid intended to be guided to the wound and/or the body cavity, in particular the pericardial cavity, to a desired infusion liquid temperature. The heater device may be part of a temperature control system of the system, which may be configured to control a temperature of the infusion liquid flow, wherein the temperature control system may accordingly comprise: a temperature sensor to measure a temperature of infusion liquid, a temperature control unit, which may be part of the control unit or a separate unit of the system and which may be configured to provide a temperature control signal on the basis of the measured temperature of infusion liquid and a desired infusion liquid temperature, and said heater device, wherein the heater device may be controlled / adjusted by the temperature control signal to heat the infusion liquid to the desired infusion liquid temperature. The temperature sensor may be arranged in or on the first tube and/or the second tube. The temperature sensor may also be configured as or in a separate element (e.g., for rectal application) being connected or connectable to the control unit and/or the temperature control unit.

The desired infusion liquid temperature may be based on a temperature of the wound and/or the body cavity, in particular the pericardial cavity. Preferably, the infusion liquid temperature is in a range of 36°C and 38°C, more preferably approximately 37°C. The desired infusion liquid temperature may also be adapted or adjusted to the actual body temperature of the patient or may be adjusted in such a manner that it heats or cools down the heart and its surroundings when clinically desired. In particular, the heating may be necessary if a patient is cooled down after an operation, which is the case in the majority of patients, and otherwise, the cooling of, e.g., the heart, which may be necessary in case of certain heart rhythm disturbances or when the patient is in a "cooling protocol" which is needed after serious adverse events peri-operatively like a period of total circulatory collapse and/or cardiopulmonary resuscitation.

The first tube and the second tube may be combined in an integrated inflow and/or outflow drain device. In this case, the infusion liquid outlet and the effusion liquid inlet may also be combined in an integrated liquid interface device being configured to connect to the integrated inflow and/or outflow drain device. For example, the first tube may be integrated in the second tube in such a way that the first tube does not consume lumen of the second tube. In particular, the first tube may be positioned in the wall or parallel to of the second tube. It may also be possible that one or more first tubes may be integrated in or on the second tube. It is preferred that the integrated inflow and/or outflow drain device should have a regular shape, such as a rounded or circular shape, in order to be placeable or placed in the patient's body without any leakage from the body cavity, in particular the pericardial cavity (and/or the pleural cavity).

The system may further comprise at least one further effusion liquid inlet to connect to at least one further tube having an effusion liquid lumen to guide the effusion liquid flow from the further body cavity or cavities, in particular the pleural cavity or the pericardial cavity, to the system. Accordingly, the at least one further effusion liquid inlet may be referred to as a second effusion liquid inlet, a third effusion liquid inlet etc. depending on the number of effusion liquid inlets, wherein the effusion liquid inlet may then be accordingly referred to as the first effusion liquid inlet. As to the tubes, the at least one further tube may be referred to as a third tube assigned to the second effusion liquid inlet, a fourth tube assigned to the third effusion liquid inlet etc. For example, the system may further comprise a second effusion liquid inlet to connect to a third tube having an effusion liquid lumen to guide the effusion liquid flow from the pericardial cavity to the system. The effusion liquid flows of the second and the third tubes may be merged by means of a fluid connector piece, such as a Y-shaped connector piece, in order to allow the liquid to be subsequently guided to the buffer container. Alternatively or additionally, the system may further comprise a third effusion liquid inlet to connect to a fourth tube having an effusion liquid lumen to guide the effusion liquid flow from the pleural cavity to the system. Optionally, the system may further comprise a fourth effusion liquid inlet to connect to a fifth tube having an effusion liquid lumen to guide the effusion liquid flow from the pleural cavity to the system. The effusion liquid flows of the fourth and the fifth tubes may be merged by means of a fluid connector piece, such as a Y-shaped connector piece, in order to allow the liquid to be subsequently guided to the buffer container or a further buffer container being particularly assigned to the liquid being received from the pleural cavity.

When the system is configured to also receive effusion liquid from the pleural cavity, the system may also comprise a further buffer container (i.e., a second buffer container), a further pump device (i.e., a third pump device), and a further hematocrit sensor (i.e., a second hematocrit sensor), wherein each of said further elements of the system are configured as outlined above with respect to the buffer container, the first pump device and the hematocrit sensor. The second buffer container, the third pump device and the second hematocrit sensor may be arranged in a parallel effusion liquid line, for example a parallel effusion liquid measurement line, assigned to the effusion liquid received from the pleural cavity and may be correspondingly arranged in the same manner as outlined above with respect to the buffer container, the first pump device and the hematocrit sensor. It may be conceivable, that this secondary system (buffer container, pump and hematocrit sensor) may be configured and/or used to separately measure the blood loss and/or air leakage.

The buffer container (and/or the second buffer container) may be configured as a cartridge or as a part of a cartridge and/or a frame construction. Optionally, the cartridge or the frame construction is disposable, in particular a disposable cartridge or a disposable frame construction. It may be conceivable, that the cartridge or the frame construction may be provided with interface elements for receiving and/or transmitting liquid and/or signals, such as control signals and/or sensor signals, from and/or to the system or a part of the system. Further, at least one of the interface elements may be configured for a power supply. Correspondingly, the system may be provided with counter interface elements corresponding to and being engageable with the interface elements of the cartridge. It may additionally or alternatively be conceivable, that the frame construction may hold or comprise one or more tubes for connecting to the counter interface elements, e.g., for connecting with pump devices, sensor devices and/or valve devices of the system.

In other words, a cartridge or frame construction, in particular a disposable cartridge or frame construction, may be provided, which may be configured to interface with the system and/or a part of the system and which may comprise the buffer container (and/or the second buffer container). Accordingly, the flushing system may be provided as described above, wherein the buffer container may be substituted with the cartridge or frame construction.

In a further embodiment, the cartridge or frame construction may also comprise the first pump device (and/or the third pump device, when the cartridge comprises the second buffer container) and/or may be engageable therewith. In a still further embodiment, the cartridge may also comprise the first pump device and the hematocrit sensor (and/or the third pump device and the second hematocrit sensor, when the cartridge comprises the second buffer container) and/or may be engageable therewith. In a still further embodiment, the cartridge or frame construction may additionally comprise the pressure sensor and/or interface and/or membrane to the pressure sensor on the belonging device. In a still further embodiment, the cartridge or frame construction may additionally comprise a lumen element assigned to the heater device and configured for guiding infusion liquid through the heater device for tempering the infusion liquid. It should be understood that connecting elements and connecting lines between the above-described elements for guiding infusion and/or effusion liquid may also be included in or on the cartridge or frame construction and may accordingly be part of the cartridge or frame construction.

It may be conceivable that said cartridge or frame construction may function as an interface between the initially-mentioned functions of the device and the inflow and outflow liquids.

It may be conceivable that the cartridge or frame construction may be mountable and/or fixable to the rest of the system by mechanical means, e.g., by clamping means and/or locking means and/or latching means. However, it may additionally or alternatively be conceivable that the cartridge or frame construction may be mountable and/or fixable to the rest of the system by vacuum means, e.g., by vacuum means sucking the cartridge or frame construction against at least a part of the rest of the system thereby reliably mounting and/or fixing the cartridge or frame construction on and/or in the system.

It may be conceivable that the integrated liquid interface device may be formed as a hub device for connecting to the integrated inflow and/or outflow drain device, e.g., having a multi-lumen formed by at least the first tube and the second tube. The hub device may be configured for at least connecting to and/or combining one or more further effusion liquid inlets, e.g., a/the second effusion liquid inlet, a/the third effusion liquid inlet and/or a/the fourth effusion liquid inlet. It may be conceivable that the hub device may comprise at least one fluid connector piece, e.g., a Y-shaped connector, for combining effusion liquid streams receivable via the corresponding effusion liquid inlets. The hub device may comprise at least one injection port for injecting additional material in the infusion liquid, such as clot dissolvent, e.g., in case of excessive clotting further downstream in the system.

It may be conceivable that the hub device may be disposable, e.g., configured as a disposable hub device.

It may be conceivable that the system comprises a hub receiving device for holding the hub device. The hub receiving device may be formed as a cradle device. The hub receiving device may include and/or house the temperature sensor of the system, e.g., to measure a/the temperature of infusion liquid, e.g., for controlling the inflow by the control unit.

It may be conceivable that the hub receiving device may be non-disposable, i.e., reusable, e.g., may be configured as a non-disposable hub receiving device.

It may be conceivable that the hub receiving device may include and/or house the heater device of the system, e.g., to heat the infusion liquid being intended to be guided to the wound and/or the body cavity, in particular the pericardial and/or pleural cavity, to a desired infusion liquid temperature.

It may be conceivable that the system comprises a slope device, wherein the slope device may be formed by the tubes of the system connecting the system to the wound and/or the body cavity, in particular the pericardial and/or pleural cavity, wherein the slope device may be formed such that said tubes are sloped from the wound and/or the body cavity, in particular the pericardial and/or pleural cavity, towards the system.

In other words, the slope device may facilitate a height difference between a patient and at least a part of the system, e.g., most of the system, (with respect to the gravity), wherein the patient may generally be arranged on a higher position.

In particular the tubes forming the slope device may be arranged such as to be sloped in a linear or circular or spiral manner, e.g., in a substantially downward oriented and meander manner and/or in a substantially downward oriented and spiral manner and/or in a substantially downward oriented and screw manner. Accordingly, a good flow in the tubing may thereby be facilitated, in particular, without sags where the fluid may possibly accumulate in the tubing.

The invention will be better understood with the aid of the description of an embodiment given by way of example and illustrated by the figures.

It is shown in
- **Fig. 1**: a flushing system according to an embodiment of the present invention;
- **Fig. 2**: a flushing system according to another embodiment of the present invention;
- **Fig. 3**: a cartridge included in the flushing system of **Fig. 2****;**
- **Fig. 4**: a flushing system according to another embodiment of the present invention;
- **Fig. 5**: a flushing system according to another embodiment of the present invention;
- **Fig. 6**: a flushing system according to another embodiment of the present invention;
- **Fig. 7**: a component of a flushing system according to another embodiment of the present invention; and
- **Fig. 8**: a top view of the component of **Fig. 7****.**

Turning to **Fig**. **1****,** a flushing system 1 configured to flush a body cavity being the pericardial cavity PC of a patient is schematically shown.

The system 1 comprises an infusion liquid outlet 2.

The infusion liquid outlet 2 is configured to connect a first tube 4.

The first tube 4 has an infusion liquid lumen to guide a flow of infusion liquid from the system 1 to the pericardial cavity PC.

The system 1 comprises an effusion liquid inlet 6.

The effusion liquid inlet 6 is configured to connect to a second tube 8.

The second tube 8 has an effusion liquid lumen to guide the effusion liquid flow from the pericardial cavity PC to the system 1.

The system 1 comprises a buffer container 10.

The buffer container 10 has a buffer volume.

The buffer container 10 comprises a buffer inlet 12 and a first buffer outlet 14.

The buffer inlet 12 is configured to receive effusion liquid from the pericardial cavity PC.

The first buffer outlet 14 is configured to output the received effusion liquid.

The system 1 comprises a flow rate control system.

The flow rate control system is configured for controlling the flow rate (e.g., a volume) of the effusion liquid output from the buffer container 10.

The flow rate control system comprises a control unit 16 and a first pump device 18.

The control unit 16 is configured to provide one or more control signals.

The first pump device 18 is configured for pumping effusion liquid from the buffer container 10 to (e.g., towards) one or more effusion liquid container 20 at an effusion liquid flow rate. The effusion liquid flow rate (e.g., the volume) is adjustable by the control signals of the control unit 16.

In the present embodiment, the first pump device 18 is a peristaltic pump. However, it is also conceivable that other kinds of pumping devices are useable.

The buffer container 10 comprises a clot trap 22.

The clot trap 22 is configured for preventing clots included in the effusion liquid being received from the pericardial cavity PC from exiting the buffer container 10 via the first buffer outlet14.

The buffer container 10 has a cylindrical shape extending along a central axis (not shown in the figures), which substantially extends along the gravitational direction. However, any other shape may be possible.

The buffer container comprises a top end 24 and a bottom end 26.

Further, the buffer container 10 comprises a top wall 28, a side wall 30 and a bottom wall 32.

The buffer inlet 12 is disposed closer to the top wall 28 than to the bottom wall 32, e.g., with respect to the gravitational direction.

The first buffer outlet 14 is disposed closer to the bottom wall 32 than to the top wall 28.

In the present embodiment, the buffer inlet 12 is arranged in the top wall 28 of the buffer container 10 and the first buffer outlet 14 is arranged in the bottom wall 32.

The flushing system 1 is configured to provide approximately 50 to 1000 ml of infusion liquid per hour, in particular approximately 500 ml of infusion liquid per hour. Hence, the buffer volume of the buffer container 10 is configured (e.g., sized) to be able to handle approximately 500 ml of effusion liquid per hour as well as sudden gushes of 400 ml or 500 ml of effusion liquid per 5 min.

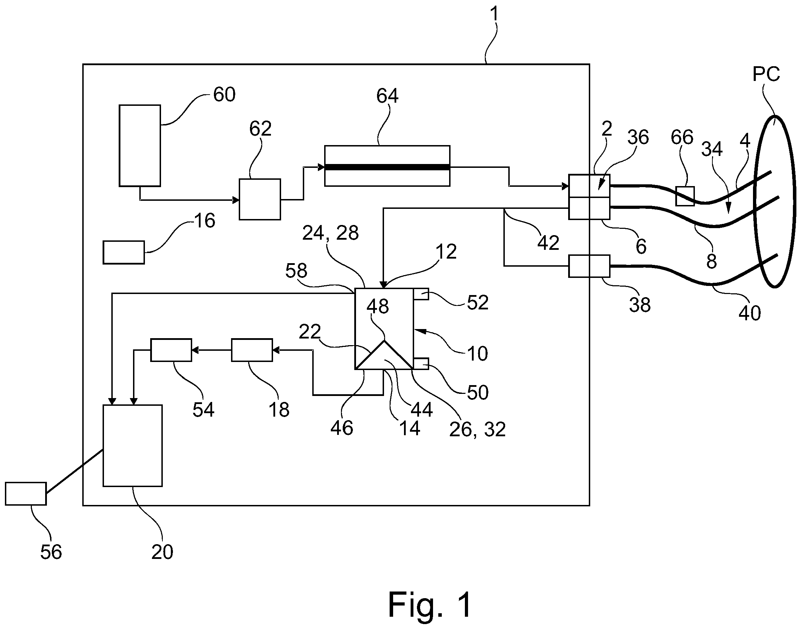

As can be seen in **Fig. 1****,** the first tube 4 and the second tube 8 are combined in an integrated inflow and/or outflow drain device 34.

As can be further seen in **Fig. 1****,** the infusion liquid outlet 2 and the effusion liquid inlet 6 are combined in an integrated liquid interface device 36, which is configured to connect to the integrated inflow and/or outflow drain device 34.

The first tube 4 is integrated in the second tube 8 in such a way that the first tube 4 does not consume lumen of the second tube 8. In the present case, the first tube 4 is positioned in the wall of the second tube 8.

Additionally, in the present embodiment, the system 1 comprises a further effusion liquid inlet 38.

The further effusion liquid inlet 38 is configured to connect a further tube 40.

The further tube 40 has an effusion liquid lumen to guide the effusion liquid flow from the pericardial cavity PC to the system 1.

In this embodiment, the effusion liquid inlet 6 can be referred to as a/the first effusion liquid inlet 6 and the further effusion liquid inlet 38 can be referred to as a/the second effusion liquid inlet 38, and the further tube 40 can be referred to as a/the third tube 40.

The effusion liquid flows of the second and the third tubes 8, 40 can be merged by means of a fluid connector piece 42. The fluid connector piece 42 may be formed as a Y-shaped connector piece.

The effusion liquid inlet 6, i.e., the integrated liquid interface device 36, is connected to the fluid connector piece 42 (e.g., via a connecting line, for example, a connecting tube).

The second effusion liquid inlet 38 is connected to the fluid connector piece 42 (e.g., via a connecting line, for example, a connecting tube).

The fluid connector piece 42 is connected to the buffer inlet 12 (e.g., via a connecting line, for example, a connecting tube).

The first buffer outlet 14 is connected to the first pump device 18 (e.g., via a connecting line, for example, a connecting tube).

In the present embodiment, the clot trap 22 is a mechanical means. In particular, the clot trap 22 comprises a sieve structure 44. The sieve structure 44 is disposed between the buffer inlet 12 and the first buffer outlet 14 of the buffer container 10. The clot trap 22 may be integrally formed with the buffer container (e.g., on the bottom wall 32 of the buffer container 10). The sieve structure 44 comprises a plurality of openings formed therein. The openings are configured (e.g., sized) to stop clots from passing through the sieve structure 44.

The clot trap 22 has a conical shape. The conical shape has a base 46 facing towards the first buffer outlet 14 and an apex 48 facing the buffer inlet 12. Accordingly, the sieve structure 44 is at least partly or completely conically shaped. It is also possible, that the clot trap may have other shapes, e.g., a truncated cone shape, a dome shape, a flat shape etc.

The buffer container 10 further comprises at least one level sensor 50, 52.

The at least one level sensor 50, 52 is configured for obtaining at least one filling level of the buffer volume.

In the present embodiment, the buffer container 10 comprises a first level sensor 50 and a second level sensor 52 for obtaining a first filling level and a second filling level of the buffer volume, respectively.

The second level sensor 52 is arranged at a higher level than the first level sensor 50 with respect to the gravitational direction.

In the present embodiment, the second level sensor 52 is arranged adjacent to the top wall 28 and the first level sensor 50 is arranged adjacent to the bottom wall 32.

However, the buffer container 10 may comprise more than said two level sensors 50, 52 in order to obtain intermediate filling levels.

Additionally or alternatively, the first level sensor 50 and/or the second level sensor 52 may also be arranged at intermediate positions between the top and the bottom wall 28, 32.

Each of the level sensors 50, 52 are configured to obtain their respective filling level by optical means, as generally known in the art. However, any other suitable sensor may also be applicable (such as mechanical sensors, capacitive sensors, resistive sensors, ultrasonic sensors etc.).

The system 1 further comprises a hematocrit sensor 54.

The hematocrit sensor 54 is configured to measure the hematocrit value of the effusion liquid for obtaining a blood loss volume and/or a blood loss flow rate from the pericardial cavity PC.

The hematocrit sensor 54 is configured to optically measure said hematocrit value based on scattering and absorption of light emitted into the effusion liquid.

The hematocrit sensor 54 comprises at least one light emitting unit and at least one light receiving unit (not shown in the figures).

As can be seen in **Fig. 1****,** the hematocrit sensor 54 is arranged at a connecting line (e.g., connecting tube) downstream from the first pump device 18.

Further downstream with respect to the hematocrit sensor 54, the one or more effusion liquid containers 20 are arranged.

The one or more effusion liquid containers 20 are connected or connectable to the connecting line, at which the hematocrit sensor 54 is arranged. However, it is also conceivable that at least two separate connecting lines are used.

In the present embodiment, as an example, the system 1 comprises (only) one effusion liquid container 20.

In this respect, the hematocrit sensor 54 is arranged downstream from the buffer container 10 and upstream from the effusion liquid container 20.

The effusion liquid container 20 is connectable or, in the present embodiment, connected to an intensive care unit (ICU) vacuum wall connector 56.

The ICU vacuum wall connector 56 can be considered as a suction device for drawing effusion liquid out of the pericardial cavity PC by means of a relative low pressure.

Said suction device, i.e., the ICU vacuum wall connector 56, can be part of the system 1 or can be assigned to the system 1 (i.e., may not explicitly be a part of the system).

The effusion liquid container 20 is accordingly connectable or, in the present embodiment, connected to the buffer container 10 via the hematocrit sensor 54, the first pump device 18 and their corresponding connecting lines, which connect each of said elements 10, 18, 20, 54. Hence, an indirect connection is provided.

Additionally, the effusion liquid container 20 has a direct connection with the buffer container 10, which is described below.

Referring again to the buffer container 10, the buffer container 10 comprises a second buffer outlet 58.

The second buffer outlet 58 is configured to output effusion liquid (e.g., surplus effusion liquid that cannot be processed timely (either the gush is too large or fast, or the system is blocked somewhere)) and/or air.

The second buffer outlet 58 is connectable or, in the present embodiment, connected to the effusion liquid container 20 (e.g., by a bypass line, for example, a bypass tube).

Hence, said direct connection may be provided between the effusion liquid container 20 and the buffer container 10, in particular, to thereby provide safety in case of any occlusion of the system by clots in the buffer container and/or occlusion of said pump and/or any lines.

The second buffer outlet 58 is arranged at a higher level than the first buffer outlet 14 and at a lower level than the buffer inlet 12, each with respect to the gravitational direction.

The second buffer outlet 58 is disposed on an opposite side of the clot trap 22 with respect to the first buffer outlet 14. Hence, the second buffer outlet 58 is disposed on the same side as the buffer inlet 12.

The second buffer outlet 58 may act as a safety overflow from the buffer container 10, to thereby provide an open connection to the effusion liquid container 20 for safety reasons (e.g., for preventing back pressure /afflux). In particular, the open connection may be understood as an always-open connection.

The second buffer outlet 58 may provide an exit path for air being separated from the effusion liquid in the buffer container 10.

Now referring to the elements of **Fig. 1** particularly disposed upstream with respect to the pericardial cavity PC.

In this regard, the system 1 comprises one or more infusion liquid containers 60.

The one or more infusion liquid containers 60 are configured for containing infusion liquid.

For example, the one or more infusion liquid containers 60 may be a number of bags containing saline fluid, such as NaCl 0,9%, as known in the art.

In the present embodiment, as an example, the system 1 comprises one infusion liquid container 60.

The system 1 comprises a second pump device 62.

The infusion liquid container 60 is connectable or, in the present embodiment, connected to the second pump device 62 (e.g., via a connecting line, for example, a connecting tube).

The second pump device 62 is configured for pumping infusion liquid to the infusion liquid outlet 2, i.e., in the present embodiment, to the integrated liquid interface device 36, at an infusion liquid flow rate. The infusion liquid flow rate is adjustable by the control signals of the control unit 16.

The second pump device 62 is a peristaltic pump. However, the second pump device 62 may be any suitable pump device for infusion liquid, that preferably allows pumping at a relative accurate flow rate.

The infusion liquid flow rate of the second pump device 62 is adjustable by the control unit 16 so that the control unit 16 can control the flow rate of infusion liquid that is pumped to the pericardial cavity PC.

The system 1 further comprises a heater device 64.

The heater device 64 is disposed downstream from the second pump device 62 and connected thereto (e.g., via a connecting line, for example, a connecting tube).

The heater device 64 is configured to heat the infusion liquid passing therethrough.

The heater device 64 may be part of a temperature control system of the system 1 (not shown in the figures). The temperature control system may be configured to control a temperature of the infusion liquid flow.

The heater device 64 is connected to the infusion liquid outlet 2, i.e., in the present embodiment, to the integrated liquid interface device 36 (e.g., by a connecting line), thereby allowing to provide the temperature controlled infusion liquid to the pericardial cavity PC.

A pressure sensor 66 is also included in the system 1 of **Fig. 1****.**

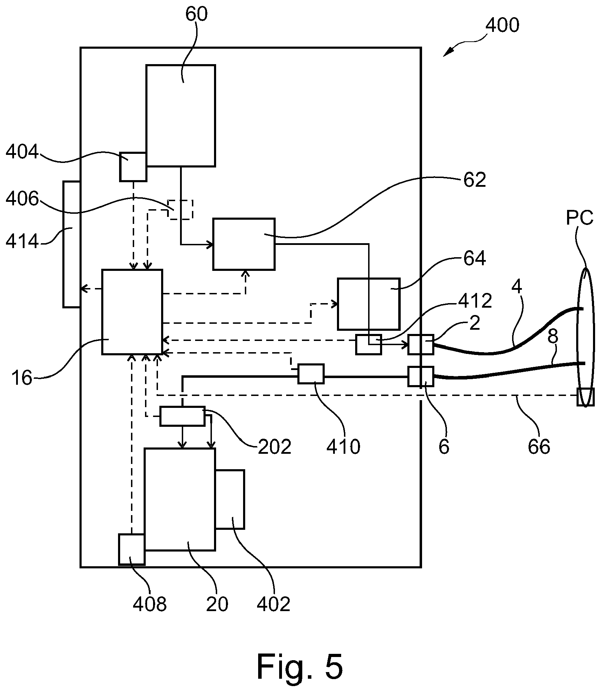

The pressure sensor 66 is provided in the first tube 4. However, the pressure sensor 66 may also be positioned in connection with the first tube 4 or inside or in connection with the second tube 8 or the pericardial cavity PC (e.g., as shown in **Fig. 5****).**

The pressure sensor 66 is configured for obtaining the pressure in the pericardial cavity PC, in particular the pressure in the space between the heart and the pericardium and/or other tissues surrounding the heart. In other words, the pressure sensor is configured to provide a pressure signal representative for the pressure in the pericardial cavity PC, in particular the pressure in the space between the heart and the pericardium and/or other tissues surrounding the heart.

Generally, the flushing system 1 is configured for continuous flushing of the pericardial cavity PC, in particularly as a post-operative treatment. During continuous flushing of the pericardial cavity PC, infusion liquid is pumped into the cavity PC to dilute blood and clots present in the cavity PC. At the same time effusion liquid, i.e. a mixture of blood and infusion liquid mixed in the pericardial cavity PC is drawn out of the cavity PC in order to remove the blood and clots from the cavity PC.

The liquid output of the pericardial cavity PC, i.e., the effusion liquid can be guided in the direction of the buffer container 10.

In particular, the effusion liquid can be guided from the pericardial cavity PC via the integrated inflow and/or outflow drain device 34.

Since the first tube 4 is integrated in the second tube 8 in such a way that the first tube 4 does not or minimally consume(s) lumen of the second tube 8 and, in particular, since the first tube 4 Isis positioned in the wall of the second tube 8 in the present exemplary case, the integrated inflow and/or outflow drain device 34 may be allowed to have a regular shape, such as a rounded or circular shape, to thereby be placeable or be placed in the patient's body without any leakage from the pericardial cavity PC. In other words, the integrated inflow and/or outflow drain device 34 may have a regular shape, such as a rounded oval, or circular shape or a multiplication of that shape.

The connection between the integrated inflow and/or outflow drain device 34 and the system is constructed in such a way that the integrated inflow and/or outflow drain device 34can be drawn through the incision of the patient from the inside to the outside, meaning that it has a connector that is not larger that the outside diameter of the integrated inflow and/or outflow drain device 34. This integrated inflow and/or outflow drain device 34 can thereafter be connected to said connector so that inflow and outflow can be established.

Said connector is outside of the system 1, so that also one or more extra chest tubes (ensuring optimal outflow from the pericardial cavity and optionally pleura or pleurae) can be connected so that the total outflow

The effusion liquid flows of the second and the third tubes 8, 40 can be merged by means of the before-mentioned fluid connector piece 42, in order to allow the effusion liquid to be subsequently guided to the buffer container 10 (e.g., by the connecting line, which connects the fluid connector piece 42 with the buffer container 10, i.e., the buffer inlet 12 of the buffer container 10).

Due to the buffer container 10, the effusion liquid is buffered before being guided further for subsequent proceedings. Further, due to the clot trap 22, the effusion liquid can be freed from clots and/or air, which may be included in the effusion liquid.

In other words, clots can be separated from the effusion liquid by means of the clot trap 22.

In particular, since the clot trap 22 comprises the sieve structure 44 being disposed between the buffer inlet 12 and the first buffer outlet 14 of the buffer container 10, clots are prevented from passing through the first buffer outlet 14.

When the system 1 is operated, each of the level sensors 50, 52 can obtain their respective filling level of the buffer volume.

The control unit 16 provides at least one of the (e.g., its) control signals on the basis of the obtained filling level of the buffer volume, to thereby control the first pump device 18 and adjust the effusion liquid rate from the buffer container. Further, this also allows to provide single liquid batches to the subsequent hematocrit sensor 54 (i.e., the hematocrit sensor, which is disposed downstream from the first pump device 18) for analysis.

In this respect, the control unit 16 is configured to control the first pump device 18 in order to pump a predetermined, i.e., a known, volume on the basis of said control signals.

For example, the control unit 16 can control the first pump device 18 in order to pump a volume of, e.g., 30 ml, which may be referred to as said batch. However, any other volume may be pumped on the basis of the control signals of the control unit 16.

Hence, the buffer container 10 collects effusion liquid until the first pump device 18 is allowed to pump.

Then, a steady flow of effusion liquid can be generated in a controlled manner. As a consequence, subsequent analysis can be performed in/at the effusion liquid in a more accurate manner.

As already described above, the first pump device 18 is a peristaltic pump, which allows pumping at a relative accurate flow rate and is configured for gently conveying of sensitive material such as blood cells, which could be destroyed by rapidly moving or rotating elements, which might be part of other kinds of pumps.

The peristaltic pump is configured to perform a continuous and/or a discontinuous pumping action (e.g., for continuous analysis or discontinuous (i.e., batch) analysis of the effusion liquid).

Said analysis may comprise measuring the hematocrit value of the effusion liquid by the hematocrit sensor 54 for obtaining a blood loss volume and/or a blood loss flow rate from the pericardial cavity PC.

As can be seen in **Fig. 1****,** the hematocrit sensor is arranged at the connecting line downstream from the first pump device 18 in order to conduct measurements in the effusion liquid pumped from the buffer container 10 (by the first pump device 18).

Due to said arrangement of the hematocrit sensor 54, precise hematocrit measurements can be conducted without negative effects of clots and/or air in the effusion liquid (due to the buffer container 10 / clot trap 22).

Further, since the first pump device 18 can be controlled with respect to the volume of liquid (i.e., volume of the liquid batches) to be pumped by the control unit 16, the measured hematocrit value can be set in correlation with the pumped volume (i.e., the batch/batches), in which the hematocrit value is measured by said sensor 54.

After passing the hematocrit sensor 54, the effusion liquid is guided to the one or more effusion liquid containers 20, i.e., in the present embodiment, to the effusion liquid container 20.

Since the second buffer outlet 58 is also connected to the effusion liquid container 20 (e.g., by the bypass line), said direct connection between the effusion liquid container 20 and the buffer container 10 is also provided thereby. Hence, effusion liquid and/or (e.g., separated) air may also be allowed to be guided therethrough.

The separated air can be (e.g., eventually) sucked into the suction device, i.e., the ICU vacuum wall connector 56, via the effusion liquid container 20 (and the bypass line).

Hence, due to the lack of air in the effusion liquid (provided by the buffer container 10), the effusion liquid can subsequently be analyzable or analyzed in a more accurate and reliable manner by the hematocrit sensor 54.

When the system 1 is in operation, before the infusion liquid may enter the patient's body or the patient's pericardial cavity PC, the infusion liquid is preferably temperature controlled to a desired infusion liquid temperature.

Accordingly, the system 1 comprises the above-described heater device 64, which, in the present embodiment, is disposed downstream from the second pump device 62.

The heater device 64 is configured to heat the infusion liquid, which is to be guided to the pericardial cavity PC, to a desired infusion liquid temperature.

The desired infusion liquid temperature is based on a temperature of the pericardial cavity PC. Preferably, the infusion liquid temperature is in a range of 36°C and 38°C, more preferably approximately 37°C. However, the desired infusion liquid temperature may also be adapted or adjusted to the actual body temperature of the patient.

Accordingly, the heater device 64 allows to provide the temperature controlled infusion liquid to the pericardial cavity PC.

Further, as described above, the pressure sensor 66 can obtain the pressure in the pericardial cavity PC. In other words, the pressure sensor 66 can provide a pressure signal representative for the pressure in the pericardial cavity PC.

The obtained pressure can be provided to the control unit 16, wherein the control unit 16 is configured to adjust the infusion liquid flow rate (by controlling the second pump device 62) to keep the pressure (i.e., for example, the pressure signal) within desired pressure limits so as to avoid acute cardiac tamponade and/or predict the emergence of acute cardiac tamponade.

In a (post)operative procedure, a patient is usually provided with two pericardial cavity drains for draining postoperative blood loss, which, with respect to the present embodiment, can be the second tube 8 and the third tube 40. The second tube 8 can be a part of the above-described integrated inflow and/or outflow drain device 34.

The infusion liquid, which is warmed to the desired infusion liquid temperature, e.g., to body temperature or, e.g., to below or above body temperature, by the heater device 64, is automatically pumped to the pericardial cavity PC through the first tube 4, which is also a part of the above-described integrated inflow and/or outflow drain device 34, by means of the second pump device 62. Thereby, the pericardial cavity PC is continuously flushed.

Generally, the standard flushing volume is 200 to 1500 ml per hour and can be adjusted if necessary. Medical staff is continuously informed on patient status through a graphical user interface (which may also be included in the system 1, e.g., in form of a display unit).

The drained fluids, i.e., the effusion liquid, which comprise(s) diluted blood and clots, are drained via the second and the third tubes 8, 40 towards the buffer container 10.

The clots and, optionally, air, which can also be part of the effusion liquid, are separated from the diluted blood liquid (which is subsequently referred to as effusion liquid) in the buffer container 10.

Subsequently, the first pump device 18 pumps batches of effusion liquid through the hematocrit sensor 54 to the effusion liquid container 20 (supported by the low pressure provided by the ICU vacuum wall connector 56).

Each batch of pumped effusion liquid is measured with respect to its hematocrit value by the hematocrit sensor 54.

Since the volume of each batch of effusion liquid is known by the control unit 16 due to known pumping properties of the first pump device 18, the blood loss can be derived from the measured hematocrit value and the corresponding batch volume, in particular in combination with the patient's own hematocrit value right after surgery.

However, it should be understood that this may not be limited to the patient's own hematocrit value right after surgery, since this may be exemplary. This is because the systemic hematocrit value of the patient may vary over time during a treatment stay, e.g., an ICU stay. This variation of the hematocrit value may be processed by the system as described herein, e.g., by the control unit 16, by allowing updates of the patient's systemic hematocrit value during the flushing treatment / therapy.

When the pericardial pressure and blood loss values or their trends reach critical values, the system 1 can give a warning to the medical staff (e.g., via a signal provided by the control unit 16, for example, via a graphical user interface and/or via an alarm, e.g., being a visual alarm, an acoustic alarm or the like) in order to adjust the postoperative procedure.

Now turning to **Fig**. **2****,** a flushing system 200 according to another embodiment is schematically shown. The flushing system 200 is substantially configured as the flushing system 1 of **Fig**. **1** described above. Hence, only the differences shall be described in the following.

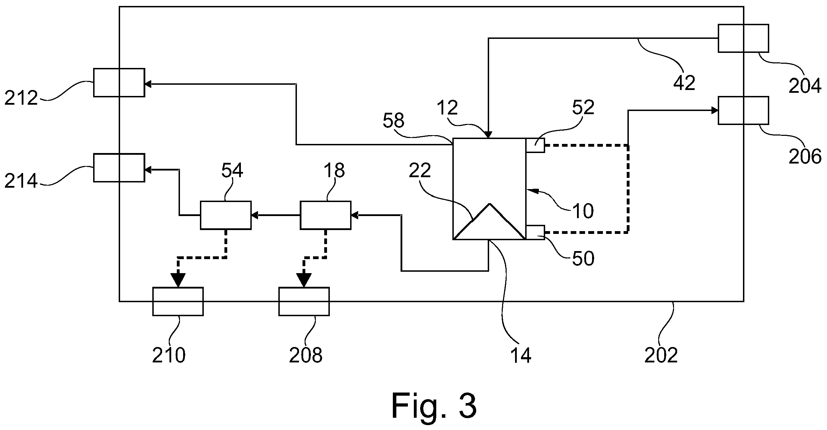

The flushing system 200 comprises a cartridge 202 (schematically depicted by dashed line in **Fig**. **2** and schematically shown in **Fig. 3****).**

The cartridge 202 is configured as a disposable cartridge, wherein the disposable cartridge is assigned to one postoperative procedure, i.e., may only be used once.

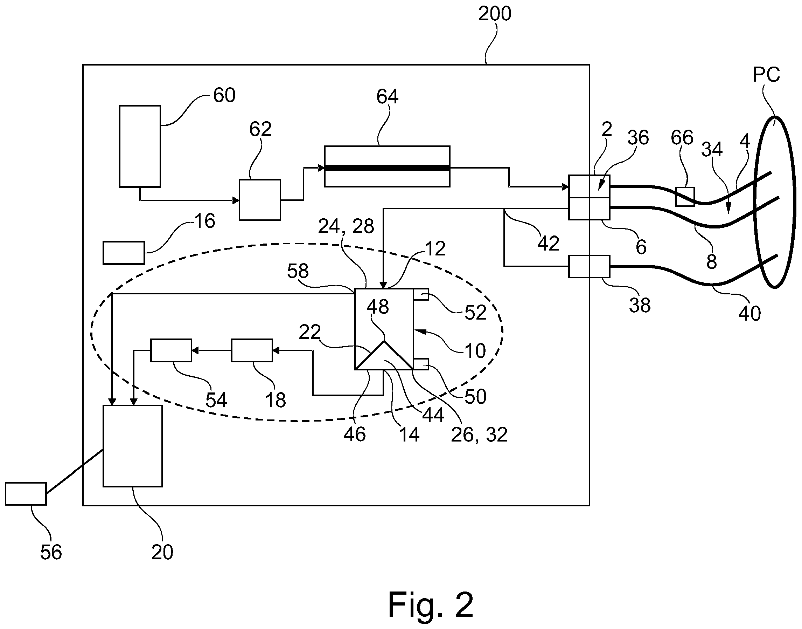

As can be seen in **Figs. 2** **and** **3****,** the cartridge 202 comprises the above-described buffer container 10, the above-described first pump device 18 and the above-described hematocrit sensor 54.

The cartridge 202 further comprises interface elements for receiving and/or transmitting liquid and/or signals, such as control signals and/or sensor signals, and/or electric power from and/or to the system 200 or a part of the system 200.

In this regard, as shown in **Fig. 3****,** the buffer inlet 12 is connectable or connected to a first interface element 204 for receiving effusion liquid.

The first and the second level sensors 50, 52 are respectively connectable or connected to a second interface element 206 (or may be each connectable or connected to separate interface elements) for power supply and/or exchanging (e.g., transmitting) sensor signals.

The first pump device 18 is connectable or connected to a third interface element 208 for power supply (e.g., electrical and/or mechanical power supply) and/or exchanging (e.g., receiving) control signals.

The hematocrit sensor 54 is connectable or connected to a fourth interface element 210 for power supply and/or exchanging (e.g., transmitting) sensor signals. Additionally or alternatively, it may be conceivable that the interface may be configured as an visual interface between the cartridge 202 and the hematocrit sensor 54 being disposed outside of the cartridge 202 and being able to optically measure through a transparent part of the cartridge 202.

It may further be conceivable, that the pressure sensor 66 may also be arranged in the cartridge 202 (not shown in the figures), wherein the pressure sensor 66 may be connectable or connected to a corresponding further interface element for power supply and/or exchanging (e.g. transmitting) sensor signals and/or pressure signals.

The second buffer outlet 58 is connectable or connected to a fifth interface element 212 for discharging effusion liquid and/or air.

Further, the cartridge 202 comprises a sixth interface element 214 for discharging effusion liquid towards the effusion container 20.

The first interface element 204 may also be configured to function as or be the integrated liquid interface device 36 in order to connect to the above-described integrated inflow and/or outflow drain device 34.

It should be understood that connecting elements and connecting lines between the above-described elements for guiding effusion liquid are also included in the cartridge 202.

It should further be understood that the system 200 also comprises counter interface elements (not shown in the figures).

The counter interface elements are configured for corresponding to the above-described interface elements of the cartridge 202.

The counter interface elements are further configured to be connectable thereto, i.e., the above-described interface elements, for the above-described purposes.

It should be further understood that the cartridge 202 is removably engageable or engaged in the system 200, which is correspondingly configured therefore.

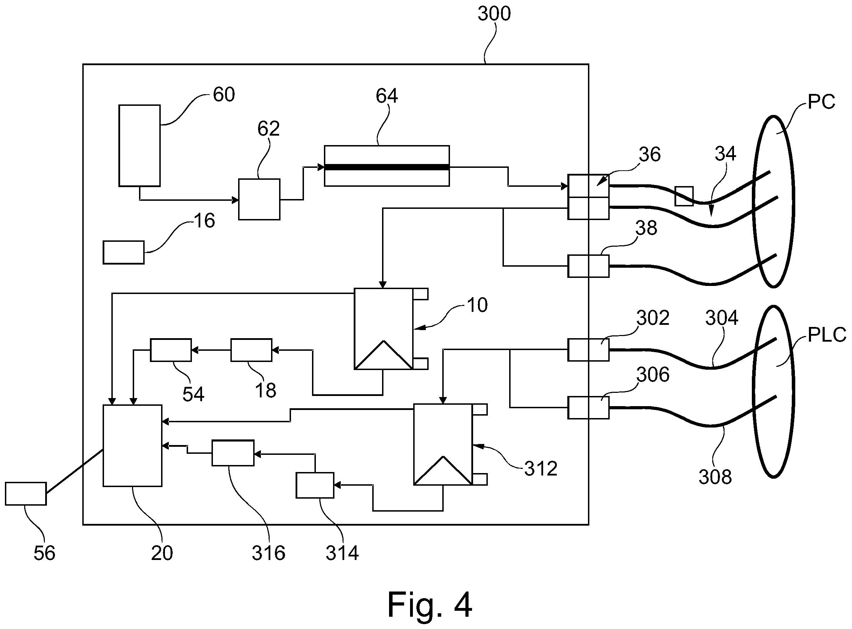

Turning to **Fig. 4****,** a flushing system 300 according to another embodiment is schematically shown. The flushing system 300 is substantially configured as the flushing system 1 of **Fig. 1** described above. Hence, only the differences shall be described in the following.

The flushing system 300 is additionally configured for draining one or two pleural cavities of the patient during flushing the pericardial cavity.

Hence, the system 300 further comprises a third effusion liquid inlet 302 and a fourth effusion liquid inlet 306.

The third effusion liquid inlet 302 is configured to connect to a fourth tube 304.

The fourth tube 304 has an effusion liquid lumen to guide the effusion liquid flow from the pleural cavity PLC to the system 300.

The fourth effusion liquid inlet 306 is configured to connect to a fifth tube 308.

The fifth tube 308 has an effusion liquid lumen to guide the effusion liquid flow from the pleural cavity PLC to the system 300.

The third effusion liquid inlet 302 and the fourth effusion liquid inlet 306 are connected to a fluid connector piece 310, such as a Y-shaped connector piece, (e.g., via a respective connecting line, for example, a respective connecting tube).

The effusion liquid flows of the fourth and the fifth tubes 304, 308 are mergeable or, when the system 300 is operated, merged by means of the fluid connector piece 310.

The system 300 comprises a further buffer container 312.

The further buffer container 312 is assigned to the liquid being received from the pleural cavity PLC.

Said further buffer container 312, which can be referred to as the second buffer container 312, is configured as the above-described buffer container 10.

The second buffer container 312 is connected to the fluid connector piece 310 (e.g., via a connecting line, for example, a connecting tube).

The system 300 also comprises a further pump device 314, i.e., a third pump device 314, and a further hematocrit sensor 316, i.e., a second hematocrit sensor 316.

Each of said further elements 312, 314, 316 of the system 300 are configured as outlined above with respect to the buffer container 10, the first pump device 18 and the hematocrit sensor 54, respectively.

The second buffer container 312, the third pump device 314 and the second hematocrit sensor 316 are arranged in a parallel effusion liquid line assigned to the effusion liquid received from the pleural cavity PLC.

The second buffer container 312, the third pump device 314 and the second hematocrit sensor 316 are correspondingly arranged in the same manner as outlined above with respect to the buffer container 10, the first pump device 18 and the hematocrit sensor 54.

Accordingly, the second buffer container 312, the third pump device 314 and the second hematocrit sensor 316 are respectively connected via a respective connecting line, for example, a respective connecting tube.

Now turning to **Fig. 5****,** a flushing system 400 according to another embodiment is schematically shown. The flushing system 400 is substantially configured as the flushing system 1 of **Fig**. **2** described above. Hence, only the differences shall be described in the following.

When the system 400 is operated, the infusion liquid flow rate of the second pump device 62 is adjustable by the control unit 16 so that the control unit 16 can control the flow rate of infusion liquid that is pumped to the pericardial cavity PC based on signals from multiple sensors.

The effusion liquid received or receivable from the pericardial cavity PC is guided or guidable to the effusion liquid containers 20.

One or more suction devices 402 are arranged next to the effusion liquid container 20.

The suction devices 402 are configured to create a relative low pressure, i.e., an under pressure of for example -15 mmHg, in the effusion liquid containers 20 (which may also be performed by an ICU vacuum wall connector 56 as configured in the other embodiments).

This relative low pressure can be used to draw effusion liquid from the pericardial cavity PC towards the effusion liquid container 20.

A weight sensor 404 is provided to measure a change in weight of the infusion liquid in the infusion liquid container 60. This change in weight is representative for a decrease in the volume of infusion liquid in the infusion liquid container 60. Based on the change of volume of infusion liquid over the course of time, the flow rate of infusion liquid towards the pericardial cavity PC may be determined.

The weight sensor 404 or the control unit 16 may be configured to determine the flow rate of infusion liquid pumped out of the infusion liquid container 60. It is remarked that any other sensor configured to determine the flow rate of infusion liquid towards the pericardial cavity PC may be used. For example, the flow rate of infusion liquid may be determined by a flow rate sensor 406 which may be located anywhere in the infusion part of the system 400. It is remarked that the flow rate of infusion liquid may also be deducted from a control signal provided by the control unit 16 to adjust the flow rate of the second pump device 62 in response to one or more of the sensor signals herein. Any other way to determine the flow rate of infusion liquid may also be used.

Effusion liquid that flows out of the pericardial cavity PC due to the suction provided by the one or more suction devices 402 is received in the effusion liquid container 20 resulting in an increase of volume of effusion liquid in this effusion liquid container 20. This increase of volume in the effusion liquid container 20 may be determined with a weight sensor 408 configured to determine a volume/weight of effusion liquid in the effusion liquid container 20. On the basis of the change of volume/weight of effusion liquid over the course of time, the flow rate/volume of effusion liquid may be determined.

Any other sensor configured to determine the flow rate of effusion liquid from the pericardial cavity PC to the effusion liquid container 20 may also be used to calculate the flow rate/volume of effusion liquid. For example, the flow rate of effusion liquid may be determined by a flow rate sensor 410.

The sensors 404, 410, the control unit 16 and the second pump device 62 may form a part of the flow rate control system to control a flow rate of the infusion liquid flow at the infusion liquid outlet 2 on the basis of the sensor signals, the sensor signals comprising an infusion liquid signal representative for an infusion liquid flow rate towards the pericardial cavity PC and an effusion liquid signal representative for an effusion liquid flow rate from the pericardial cavity PC. The difference between the infusion liquid flow rate and the effusion liquid flow rate may generally correspond substantially to the blood loss flow rate of the patient from the pericardial cavity PC. Importantly, the control signals also comprise the hematocrit sensor signal from the hematocrit sensor 54 and the pressure signal from the pressure sensor 66 placed in the pericardial cavity PC.

On the basis of the calculated blood loss volume and/or flow rate, a suitable infusion liquid flow rate can be pumped by the second pump device 62.

Since it is important to accurately determine the actual blood loss volume from the pericardial cavity PC, the system 400 also comprises the above-described cartridge 202.

Accordingly, the above-described elements 10, 18, 54 are also included therein. However, it should be understood that the respective constituents 10, 18, 54 of the cartridge 202 may each be included in the system 400 as separate elements.

The hematocrit sensor 54 of the cartridge 202 generates a blood flow signal representative for the relative amount of blood or composition of the blood in the effusion liquid. The actual blood loss may be calculated from the outflow volume (e.g., outflow volumes; e.g., as described above and/or from the buffer container 10) and the hematocrit sensor values of the blood flow. This blood flow signal is provided as an input to the control unit 16 in order to control, e.g., automatically stop, the infusion flow rate of infusion liquid pumped towards the pericardial cavity PC. Said automatic stop to a/the respective pump device may be provided, e.g., in case of too high blood loss.

In the present embodiment, the system 1 comprises a temperature control system comprising a temperature sensor 412 to measure a temperature of infusion liquid, the control unit 16 to provide a temperature control signal on the basis of the measured temperature of infusion liquid and a desired infusion liquid temperature, and the above-described heater device 64 that is controllable by the temperature control signal to heat the infusion liquid to the desired infusion liquid temperature. It is remarked that the control of the temperature control system does not have to be integrated in the control unit 16, but may also be formed by a separate control unit, for example a part of the heater device 64, or may be integrated in another control device or processing unit.

Since the control unit 16 can determine a blood loss volume or blood loss flow rate from the pericardial cavity PC of a patient based on input from the hematocrit sensor 54, this determined, in particular calculated, blood loss volume or blood loss flow rate may be used to monitor the blood loss from the pericardial cavity PC. In particular, the control unit 16 can determine a blood loss volume or blood loss flow rate from the pericardial cavity PC of a patient based on input of the hematocrit sensor 54 in combination with volume of the liquid, to which the hematocrit measurement is applicable, and with the systemic hematocrit value of the patient right after surgery.

For example, the determined blood loss volume or blood loss flow rate may be displayed by a graphical user interface, e.g., a display device 414, so that a physician or nurse can easily monitor the development of the blood loss over time. Such display device 414 may also be used to display any other relevant parameter of the flushing process, such as used volume of infusion liquid, volume of received effusion liquid, flow rates of infusion liquid and effusion liquid, relative blood content in effusion liquid, composition of blood in effusion liquid, and trends thereof. Importantly, the display may show instructions based on the combined sensor input as explained elsewhere. It should be understood that the before-mentioned may also be implemented in the embodiments shown in the figures 1 to 4.

The blood loss volume, blood loss flow rate or other relevant parameters may also automatically be monitored by an alarm device, for example integrated in control unit 16, that issues an alarm signal when the blood loss volume and/or blood loss flow rate and/or other parameters exceeds a threshold value. The alarm signal may be any suitable signal such as a visible or an audible signal. It is remarked that instead of control unit 16, any other (processing) unit or device may also be used to determine/calculate the blood loss volume or blood loss flow rate based on input from the hematocrit sensor. It should be understood that the before-mentioned may also be implemented in the embodiments shown in figures 1 to 4.

In view of the embodiment of **Fig**. **5****,** it should be understood that any element described therewith may also be integrated, used and/or included in connection with the embodiments of shown in **Fig**. **1 to 4****,** such as the sensor units in connection with the control unit being correspondingly configured, the display device, the alarm device etc.

Now turning to **Fig**. **6****,** a flushing system 500 according to another embodiment is schematically shown. The flushing system 500 is substantially configured as the flushing systems 1, 200, 300, 400 described above. Hence, only the differences shall be described in the following.

It should be understood that, in view of the embodiment of **Fig**. **6****,** any element described therewith may also be integrated, used and/or included in connection with the embodiments of shown in **Fig**. **1 to 5****,** and vice versa.

The flushing system 500 substantially comprises a base construction 502 and a frame construction 504.

The frame construction 504 is formed as a disposable frame construction.

The base construction 502 is formed as a mobile base construction being movable over the ground.

The base construction 502 comprises a bottom portion 506 and a top portion 508.

The infusion liquid container 60, here, as an example, four saline bags, and the effusion liquid container 20 are disposed and arranged in the bottom portion 506.

Further, the suction device 402 is also disposed and arranged in the bottom portion 506.

The control unit 16, the first pump device 18, the second pump device 62 and the hematocrit sensor 54 are disposed and arranged in and/or on the top portion 508.

Further, a first valve 510 and a second valve 512 are disposed and arranged in and/or on the top portion 508.

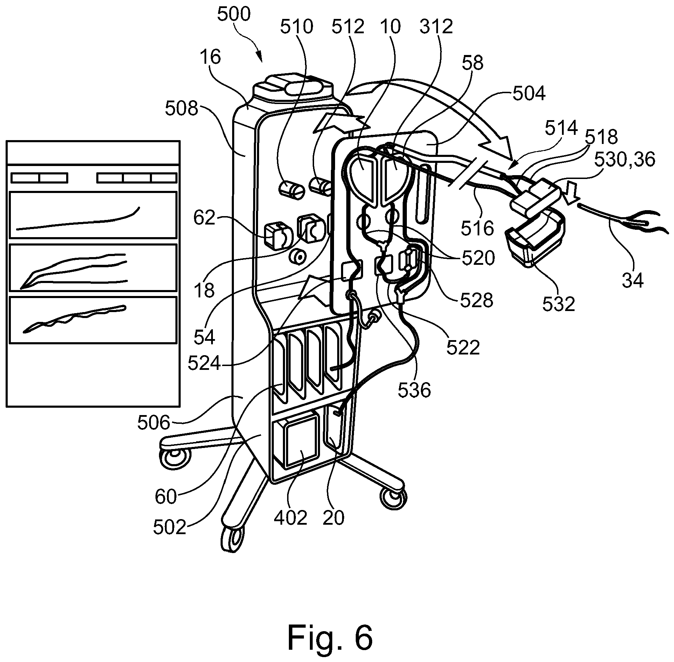

The top portion 508 is configured to hold and/or fix the frame construction 504 onto the base construction 502 in a removable and/or exchangeable manner (depicted by corresponding arrows in Fig. 6). It may be conceivable that the frame construction 504 may be mountable and/or fixable to the rest of the system, here, the base construction 502, by mechanical means, e.g., by clamping means and/or locking means and/or latching means (not shown in the figure), as known in the art.

The buffer container 10 and the second buffer container 312 are configured as a part of the frame construction 504.

In other words, the buffer container 10 and the second buffer container 312 are disposed on and are integrated in the frame construction 504.

As can be seen in **Fig. 6****,** the frame construction 504 holds and/or comprises one or more connecting lines, e.g., connecting tubes 514.

In particular, the integrated liquid interface device 36 is assigned to the frame construction 504 and is connected thereto by said connecting tubes 514, wherein one infusion connecting tube 516 is assigned to infusion liquid and two effusion connecting tubes 518 are assigned to effusion liquid.

One effusion connecting tube 518 is connected to the buffer container 10 and the other effusion connecting tube 518 is connected to the second buffer container 312.

The infusion connecting tube 516 connects the infusion liquid container 60 with the integrated liquid interface device 36 via the frame construction 504.

Downstream form the buffer containers 10, 312 and originating therefrom, further effusion connecting tubes 520 are merged in one still further effusion connecting tube 522 by means of a fluid connector piece, e.g., a Y-shaped connector.

Said still further effusion connecting tube 522 is then/further downstream merged with a connecting tube originating from the second buffer outlet 58 of the buffer containers 10, 312 by means of another fluid connector piece, e.g., another Y-shaped connector, before terminating in and/or at the effusion liquid container 20.

The frame construction 504 at least comprises a first, a second and a third access element 524, 526, 528.

The first access element 524 is configured for engaging with the second pump device 62 such that the infusion connecting tube 516 is engageable with the second pump device 62 for performing a peristaltic pumping action.

The second access element 526 is configured for engaging with the first pump device 18 such that the still further effusion connecting tube 522 is engageable with the first pump device 18 for performing a peristaltic pumping action.

The third access element 528 is configured for engaging with the hematocrit sensor 54 such that the still further effusion connecting tube 522 is engageable with the hematocrit sensor 54 for performing hematocrit measurements.

The still further effusion connecting tube 522 is engageable with the hematocrit sensor 54 further downstream with respect to the first pump device 18.

The frame construction 504 also comprises two further recesses disposed between the buffer containers 10, 312 and the fluid connector piece merging the further effusion connecting tubes 520.

Said further recesses are configured for respectively engaging the first valve 510 and the second valve 512 such that the first valve 510 is engageable with the further effusion connecting tube 520 from the buffer container 10 and such that the second valve 512 is engageable with the further effusion connecting tube 520 from the second buffer container 312.

In particular, switching between the buffer containers 10, 312 is thereby facilitated, e.g., for focusing the measurements of the hematocrit sensor 54 to only one effusion liquid of the buffer container 10 or the second buffer container 312, which, e.g., may in turn allow measurement switching between effusion liquid from the pericardial or the pleural cavity PC, PLC.

As can be seen in **Fig. 6****,** the integrated liquid interface device 36 is formed as a hub device 530 for connecting to the integrated inflow and/or outflow drain device 34.

The hub device 530 combines two or more effusion liquid inlets, e.g., being respectively assignable to the wound and/or the body cavity, in particular the pericardial and/or pleural cavity PC, PLC.

The system 500 further comprises a hub receiving device 532 for holding the hub device 530.

The hub receiving device 532 is formed as a cradle device.

The hub receiving device 532 includes and/or houses the temperature sensor 412 of the system 500, e.g., to measure a/the temperature of infusion liquid, e.g., for controlling the inflow by the control unit 16.

Further, the hub receiving device 532 includes and/or houses the heater device 64 of the system 500, e.g., to heat the infusion liquid being intended to be guided to the wound and/or the body cavity, in particular the pericardial and/or pleural cavity PC, PLC, to a desired infusion liquid temperature.

It should be understood that the flushing system 500 substantially operates in the same way as the flushing systems 1, 200, 300, 400 described further above.

The flushing system 500 provides a compact and mobile solution for flushing a wound and/or a body cavity, which may allow a flexible use in a clinical routine.

In particular, the flushing system 500 provides a compact and/or cost effective solution comprising only one pump device 18 for effusion liquid and one hematocrit sensor 54, while still being able to measure effusion liquid from different origins, e.g., the pericardial and the pleural cavity, individually.

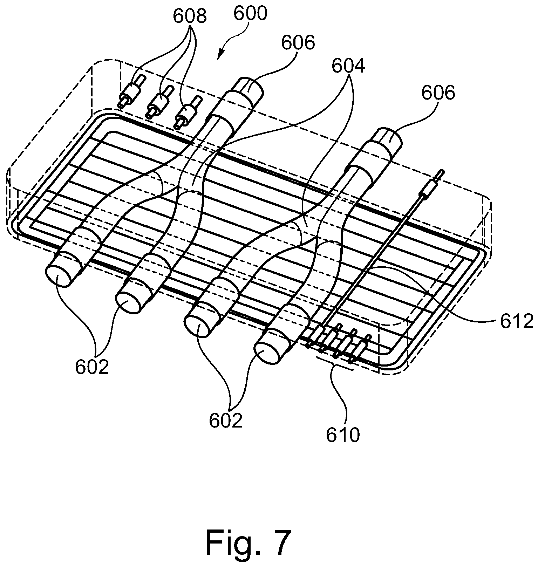

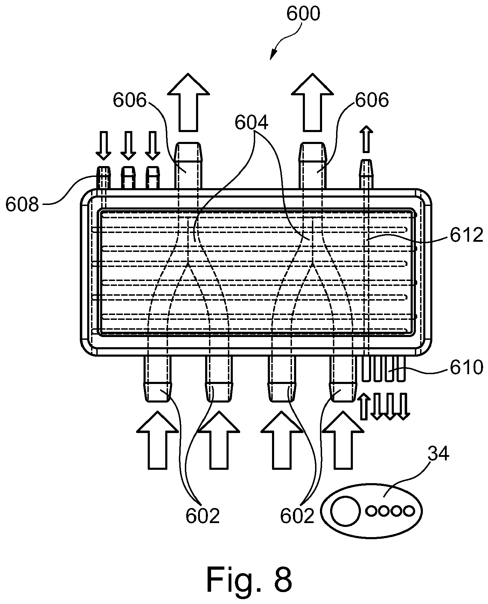

Now turning to **Figs. 7** **and** **8****,** a component, in particular a hub device 600, of a flushing system according to another embodiment of the present invention is schematically shown. The hub device 600 could be used, for example, in the flushing system 300 of **Fig. 4****.**

The hub device 600 is substantially cuboid-shaped. However, any other shape may be conceivable.

The hub device 600 combines four effusion liquid inlets 602 being disposed on one side, e.g., the patient side, of the hub device 600.

The hub device 600 comprises two fluid connector pieces 604 being disposed in the hub device 600 and being formed as Y-shaped connectors for respectively combining two effusion liquid streams receivable via the corresponding effusion liquid inlets 604.

The hub device 600 comprises two outlets 606 for letting out the merged effusion liquid streams.

The outlets 606 are disposed on the opposite side of the hub device 600, e.g. the system side, in particular, the side of the frame construction 504, (with respect to the one side, e.g., the patient side).

Further, the hub device 600 comprises one or more infusion inlets 608 on the system side for receiving infusion liquid and one or more infusion outlets 610 on the patient side for letting out infusion liquid.

Additionally, the hub device 600 comprises a measurement channel 612, e.g., for air, having an inlet on the patient side and an outlet of the system side. The measurement channel 612 may serve for pressure measurements. Hence, there may be no pressure noise from the pump devices, no risk of height difference effect in the fluid columns (e.g., compared to pressure measurement in, e.g., the effusion liquid) and additionally it may be a cost sensitive implementation.

It should be understood that the patient side of the hub device 600 is configured for connecting to the integrated inflow and/or outflow drain device 34 and that, accordingly, the integrated inflow and/or outflow drain device 34 is correspondingly configured to connecting to the patient side of the hub device 600, at least partly as shown in Fig. 8 (see cross section of the integrated inflow and/or outflow drain device 34).

It should be understood that the system, in which the hub device 600 is usable, comprises a tube arrangement corresponding to the system side of the hub device 600.

It should be further understood that the arrows depicted in **Fig. 8** indicate the respective stream direction of the corresponding fluids to be guided therethrough.

### Reference Signs

- 1: flushing system
- 2: infusion liquid outlet
- 4: first tube
- 6: effusion liquid inlet, first effusion liquid inlet
- 8: second tube
- 10: buffer container, first buffer container
- 12: buffer inlet
- 14: first buffer outlet
- 16: control unit
- 18: first pump device
- 20: effusion liquid container
- 22: clot trap
- 24: top end of buffer container
- 26: bottom end of buffer container
- 28: top wall of buffer container
- 30: side wall of buffer container
- 32: bottom wall of buffer container
- 34: integrated inflow and/or outflow drain device
- 36: integrated liquid interface device
- 38: further effusion liquid inlet, second effusion liquid inlet
- 40: further tube, third tube
- 42: fluid connector piece
- 44: sieve structure
- 46: base of clot trap
- 48: apex of clot trap
- 50: first level sensor
- 52: second level sensor
- 54: hematocrit sensor
- 56: intensive care unit (ICU) vacuum wall connector
- 58: second buffer outlet
- 60: infusion liquid container
- 62: second pump device
- 64: heater device
- 66: pressure sensor

- 200: flushing system
- 202: cartridge
- 204: first interface element
- 206: second interface element
- 208: third interface element
- 210: fourth interface element
- 212: fifth interface element
- 214: sixth interface element

- 300: flushing system
- 302: third effusion liquid inlet
- 304: fourth tube
- 306: fourth effusion liquid inlet
- 308: fifth tube
- 310: fluid connector piece
- 312: further buffer container, second buffer container
- 314: further pump device, third pump device
- 316: further hematocrit sensor, second hematocrit sensor

- 400: flushing system
- 402: suction device
- 404: weight sensor
- 406: flow rate sensor
- 408: weight sensor
- 410: flow rate sensor
- 412: temperature sensor
- 414: display device

- 500: flushing system
- 502: base construction
- 504: frame construction
- 506: bottom portion
- 508: top portion
- 510: first valve
- 512: second valve
- 514: connecting tube
- 516: infusion connecting tube
- 518: effusion connecting tube
- 520: further effusion connecting tube
- 522: still further effusion connecting tube
- 524: first access element
- 526: second access element
- 528: third access element
- 530: hub device
- 532: hub receiving device

- 600: hub device
- 602: effusion liquid inlet
- 604: connector piece
- 606: outlet
- 608: infusion inlet
- 610: infusion outlet
- 612: measurement channel

- PC: pericardial cavity
- PLC: pleural cavity

## Claims

1. A flushing system (1, 200, 300, 400, 500) configured to flush a wound and/or a body cavity, in particular the pericardial cavity (PC), of a patient, wherein the system (1, 200, 300, 400, 500) comprises:
- an infusion liquid outlet (2) to connect a first tube (4) having an infusion liquid lumen to guide a flow of infusion liquid from the system (1, 200, 300, 400, 500) to the wound and/or the body cavity, in particular the pericardial cavity (PC),
- an effusion liquid inlet (6) to connect to a second tube (8) having an effusion liquid lumen to guide the effusion liquid flow from the wound and/or the body cavity, in particular the pericardial cavity (PC), to the system (1, 200, 300, 400, 500),
- a buffer container (10) having a buffer volume and comprising a buffer inlet (12) to receive effusion liquid from the wound and/or the body cavity, in particular the pericardial cavity (PC), and a first buffer outlet (14) to output the received effusion liquid,
- a flow rate control system to control the flow rate of the effusion liquid output from the buffer container (10), wherein the flow rate control system comprises:
▪ a control unit (16) to provide one or more control signals,
▪ a first pump device (18) for pumping effusion liquid from the buffer container (10) to one or more effusion liquid container (20) at an effusion liquid flow rate, wherein the effusion liquid flow rate is adjustable by the control signals of the control unit (16),
- wherein the buffer container (10) comprises a clot trap (22) configured for preventing clots included in the effusion liquid being received from the wound and/or the body cavity, in particular the pericardial cavity (PC), from exiting the buffer container (10) via the first buffer outlet (14), and/or wherein the buffer container (10) is configured for air-liquid-separation, and/or configured with a safety bypass, and/or configured for receiving liquid gushes.

2. The system (1, 200, 300, 400, 500) of claim 1,
**characterized in that**
the clot trap (22) comprises a sieve structure (44) being disposed between the buffer inlet (12) and the first buffer outlet (14) of the buffer container (10), to thereby prevent clots from passing through the first buffer outlet (14).

3. The system (1, 200, 300, 400, 500) of claim 1 or 2,
**characterized in that**
the clot trap (22) has a conical shape having a base (46) facing towards the first buffer outlet (14) and an apex (48) facing the buffer inlet (12).

4. The system (1, 200, 300, 400, 500) of any of the preceding claims,
**characterized in that**
the buffer container (10) further comprises at least one level sensor (50, 52) for obtaining at least one filling level of the buffer volume.

5. The system (1, 200, 300, 400, 500) of claim 4,
**characterized in that**
the buffer container (10) further comprises a first level sensor (50) and a second level sensor (52) for obtaining a first filling level and a second filling level of the buffer volume, respectively, wherein the second level sensor (52) is arranged at a higher level than the first level sensor (50) with respect to the gravitational direction.

6. The system (1, 200, 300, 400, 500) of claim 4 or 5,
**characterized in that**
the control unit (16) provides at least one of the control signals on the basis of the obtained filling level of the buffer volume and/or a buffer container level, to thereby control the first pump device (18) and adjust the effusion liquid rate from the buffer container (10).

7. The system (1, 200, 300, 400, 500) of any of the preceding claims,
**characterized in that**
the first pump device (18) comprises a peristaltic pump.

8. The system (1, 200, 300, 400, 500) of any of the preceding claims,
**characterized in that**
the system further comprises one or more effusion liquid containers (20) to receive effusion liquid out of the buffer container (10).

9. The system (1, 200, 300, 400, 500) of any of the preceding claims,
**characterized in that**
the buffer container (10) further comprises a second buffer outlet (58) being arranged at a higher level than the first buffer outlet (14) with respect to the gravitational direction and being configured to output effusion liquid and/or air, to thereby allow an air-liquid-separation and/or allow to function as the safety bypass, optionally, wherein the second buffer outlet (58) is disposed on an opposite side of the clot trap (22) with respect to the first buffer outlet (14).

10. The system (1, 200, 300, 400, 500) of any of the preceding claims,
**characterized in that**
the system further comprises a hematocrit sensor (54) configured to measure the hematocrit value of the effusion liquid for obtaining a blood loss volume and/or a blood loss flow rate from the wound and/or the body cavity, in particular the pericardial cavity (PC).

11. The system (1, 200, 300, 400, 500) of claim 10,
**characterized in that**
the hematocrit sensor (54) is disposed for conducting measurements in the effusion liquid pumped from the buffer container (10) to the one or more effusion liquid containers (20).

12. The system (1, 200, 300, 400, 500) of claim 10 or 11,
**characterized in that**
the system (1, 200, 300, 400) further comprises and/or is connectible to a unit for blood loss measurements and/or patient systematic measurements, which is configured for obtaining the blood loss volume and/or the blood loss flow rate from the wound and/or the body cavity, in particular the pericardial cavity (PC).

13. The system (1, 200, 300, 400, 500) of any of the preceding claims,
**characterized in that**
the system further comprises a second pump device (62) for pumping infusion liquid to the infusion liquid outlet (2) at an infusion liquid flow rate, wherein the infusion liquid flow rate is adjustable by the control signals of the control unit (16), optionally, wherein the second pump device (62) comprises a peristaltic pump.

14. The system (1, 200, 300, 400, 500) of any of the preceding claims,
**characterized in that**
the system further comprises a pressure sensor (66) being positioned inside or in connection with the first tube (4), the second tube (8), the buffer container (10) or the wound and/or the body cavity, in particular the pericardial cavity (PC), and being configured for obtaining the pressure in the wound and/or the body cavity, in particular the pericardial cavity (PC).

15. The system (1, 200, 300, 400, 500) of any of the preceding claims,
**characterized in that**
the system further comprises a heater device (64) configured to heat the infusion liquid being intended to be guided to the pericardial cavity (PC) to a desired infusion liquid temperature, optionally, wherein the desired infusion liquid temperature is based on a temperature of the pericardial cavity (PC).

16. The system (1, 200, 300, 400, 500) of any of the preceding claims,
**characterized in that**
the first tube (4) and the second tube (8) are combined in an integrated inflow and/or outflow drain device (34).

17. The system (1, 200, 300, 500) of any of the preceding claims,
**characterized in that**
the system further comprises at least one further effusion liquid inlet (38, 302, 306) to connect to at least one further tube (40, 304, 308) having an effusion liquid lumen to guide the effusion liquid flow from the pleural cavity (PLC) or the pericardial cavity (PC) to the system (1, 200, 300, 400, 500).
